# EUROPEAN PATENT SPECIFICATION

(11) **EP 0 345 897 B1**
(45) Date of publication and mention of the grant of the patent: **08.11.1995**
(21) Application number: 89201439.0
(22) Date of filing: 05.06.1989
(51) Int. Cl.: G01N 33/543, G01N 33/548

(54) **Novel heterogeneous immunoassay**
Heterogener Immuntest
Immunoessai hétérogène

(30) Priority: 06.06.1988 US 203483
(43) Date of publication of application: 13.12.1989
(73) Proprietor: ORION CORPORATION, Orion, 02101 Espoo (FI)
(72) Inventor: Henry, Robert, F-75015 Paris (FR); Bailey, Anne, Wayne, NJ 07470 (US)
(74) Representative: de Bruijn, Leendert C.

(56) References cited:
- EP-A- 0 253 270
- WO-A-86/06491
- FR-A- 2 277 563
- GB-A- 2 017 909
- GB-A- 2 138 131
- US-A- 3 970 429

## Description

### BACKGROUND OF THE INVENTION

### Field of the Invention

This invention relates to immunodiagnostic assay techniques and to methods for providing reagents which operate in an immunoassay in a more convenient manner than conventional techniques. Specifically, it relates to the provision of certain reagents in the form of solid tablets and the use of diluents to aid in releasing unbound labeled reactants from the solid-phase.

### Description of Prior Art

Heterogeneous immunoassay procedures are based on the most versatile immunoassay technology, covering the best range of sensitivity as well as the widest range of analyte size for clinical testing.

Other technologies, such as the EMIT technology, or fluorescence polarization have shown the potential for test procedures easy to use on automated systems. These so-called homogeneous procedures have limitations for sensitivity and analyte size that heterogeneous procedures do not have.

A heterogeneous procedure currently uses a solid-phase separation step. A solid-phase, either a hard plastic component like a large bead or a tube, or a particulate support like an agarose gel, is covalently bound to an antibody or to an antigen analog. This solid-phase is used to make a separation of the two fractions resulting from the antigen-antibody reaction, the "free" fraction and the "bound" fraction.

Most of the existing heterogeneous procedures use enzyme labels like the ELISA technology, and involve multiple reagents addition and multiple washing steps. They are complex and labor-intensive.

In order to simplify heterogeneous procedures, the art has adopted procedures which can apply to small and large analytes with the same steps.

For large analytes, the method of choice is the "sandwich" procedure. A solid-phase is bound to an antigen-specific antibody and a second antigen-specific antibody is labeled. The procedure consists of mixing the sample with the solid-phase and the label either simultaneously or at different times. For small analytes, a seldom used competitive procedure has been chosen because its steps are similar to those of the sandwich procedure.

It uses a labeled antibody and a solid-phase on which is covalently bound an antigen analog. These two components can either be separate, or be reacted together, in such a way that the analyte will displace the labeled antibody bound to the solid-phase. The antibody which is not bound to the analyte is captured by the solid-phase. The solution contains the antibody bound to the analyte. In both cases (small and large analytes) the first step is to react the analyte with the labeled antibody, then to allow the solid-phase to capture either the free antibody (small analyte) or the bound antibody (large analyte). Addition of the two reagents to the sample can also be done simultaneously. Ultimately, in each case the unbound labeled molecule must be separated from the bound labeled molecule in order to assay the amount of binding which occurs. This then is a measure of the amount of antigen originally present in the sample.

EP-A-253270 discloses an immunoassay method of the above type wherein, after the labeled antibody has been contacted with the analyte and before the immunocomplex is measured, a solid phase is added to remove excess labeled antibody.

GB-A-2017909 discloses a solid-phase support for radioimmunoassay columns which swells upon contact with an antigen-antibody-containing solution after incubation of the latter.

### Summary of the Invention

The purpose of the present invention is to simplify the heterogeneous procedures described above by providing reagents which are simpler to use while in heterogeneous procedures, and which result in a faster, easier method of separation. In the heterogeneous immunoassay according to the invention a sample containing an analyte is contacted with a labeled antigen or antibody in the presence of said solid phase to produce an immunological complex on said solid-phase and an unbound labeled antigen or antibody, said solid phase is provided in the form of a tablet and the thus contacted solid phase is mixed in a diluent in which the unreacted antigen or antibody is soluble.

The present invention accordingly, provides the immunoassay reagents which are used in a heterogeneous assay in the form of a tablet and utilizes at least one, and preferably two diluents, optionally one for sample predilution and the other for solid-phase treatment after the immunological reaction. The immunological reagents, such as the dye-labeled antibody and the solid-phase carrying appropriate antibody thereon are lyophilized and tableted using standard lyophilization and tableting techniques. The reagents are used by adding the sample, preferably prediluted with diluent, and the tablet to a first tube, incubating and then connecting the first tube to a second tube to be described below forming a single assembly. The contents are mixed, the solid-phase allowed to settle, and diluent added to the solid-phase to aid in the liberation of the unbound labeled reagent into the supernatant. The absorbance of the dye is then read in an appropriate photometer.

Any components normally used in immunoassays may be employed, such as the antibodies, antigens and solid-phases. A chromogenic substance, i.e., a dye with an absorbing light in the wavelength range of 450 nm to 650 nm is most suitable as the label in the present invention. Because the molecular extinction coefficient of any dye is not high enough to allow the reading sensitivity that many of the immunoassay tests require, many of the tests will involve the use of polymeric dyes. Such polymeric dyes have been already described in U.S. Patent No. 4,452,886.

Dyes directly bound to antibodies may be used in low sensitivity tests. The use of such dye labels allows the elimination of the two last steps of an enzyme-label assay: the color development and the stopping of the reaction. Dyes such as fluorescein, Congo Red, Chicago Sky Blue and the like are preferred.

In accordance with the present invention, the reagents are tableted, preferably in a single tablet, sometimes in two. These reagents are the labeled antibody and the solid-phase about which more will be described below. Effervescent additives such as sodium bicarbonate and citric acid are added to the tablet to help dissolution of the tablet without shaking. Other additives such as fillers, binders and lubricants common in tableting are also used.

The solid-phase materials may be any normally used in the immunoassay field provided they can be tableted and are in a form which when liberated from the tablet settle from the liquid phase within a few minutes. We have discovered that swellable gels are most suitable with agarose gels being preferred. Such gels in the form of fairly large beads of the order of 150 to 300 µm in diameter and preferably 175-225 µm can be lyophilized and then compressed in a tablet form along with other reagents. When placed in a liquid medium, the tablet dissolves or effervesces, the gel swells and during this swelling the antigen-antibody reaction occurs facilitated by the absorption of the liquid into the gel.

The reaction results in an antigen-antibody solid-phase complex, and free labeled material within the interstitial liquid. To make the separation, the invention utilizes the addition of the buffered diluent described below to the gel in the reaction tube with mixing. The large beads settle by gravity within a few minutes resulting in a compact solid gel at the bottom of the tube. Above this gel is a solution with the unbound labeled antibody which can be measured directly in a photometer where the light beam is well above the bottom of the tube thus measuring only the effect of the label in solution. This permits the assay to be conducted without making a physical separation of the solid-phase out of the tube.

The diluents in the present invention are those which facilitate the liberation of the unbound labeled antibody (or in appropriate cases unbound labeled antigen) from the solid-phase after reaction. It has been discovered that an especially suitable material for this purpose is a Tris buffer and particularly one at a pH of 8.5 containing 0.15 Molar Tris, 0.15 Molar NaCl, 0.1% bovine serum albumin and 0.1% sodium azide.

The techniques of tableting and lyophilization are those normally employed in the art. For example, the lyophilization is conveniently conducted at -45^{o}C under appropriate vacuum. The art will instantly appreciate the techniques suitable which may be modified according to the particular reagents employed in the immunoassay.

In order to further simplify the handling of these procedures by non-skilled persons, all reagents are presented in a kit with the following components:
a. A reaction tube contains a diluent buffer for the sample (usually serum or plasma). The volume of this buffer is compatible with the quantity of agarose gel in the tablet.
b. A tablet (sometimes two tablets, which can be bound together) which contains all active reagents: the solid-phase and the labeling molecule.
c. A reading tube which contains the diluent buffer needed in the last step of the procedures.

In practice, the use of these components is as follows, and is common to a sandwich procedure and to a competitive procedure:
1. The sample is added to the reaction tube and shaken in order to homogenize. A tablet is dropped in the tube which is then incubated preferably at controlled temperature (room temperature to about 37^{o}C).
2. When this incubation is finished, the reaction tube is connected to the diluent tube. They are designed to form a liquid-tight tube assembly. This tube assembly is reversed three times or more in order to homogenize the slurry. Then the tube assembly is allowed to rest until the beads have settled, the reading tube being at the bottom. The time needed to let the gel settle is generally less than 5 minutes, usually and preferably less than 2 minutes. The tube assembly can then be placed in a photometer which can read above the gel. If the wavelength of the dye label is above 450 nm, more preferably above 500 nm, serum components which are still present in the reading tube do not interfere significantly, providing the serum or plasma sample volume does not exceed 50 microliters. A bichromatic photometer is preferred to other optical devices in order to make this reading more free from these interferences. A wavelength references of 600 nm is preferred when the dye-wavelength is 500 nm.

The following examples are provided to illustrate preferred embodiments of the invention.

### Example 1

### Use of dye-labeled antibody and tableted reagents in a simultaneous sandwich assay.

A sandwich immunoassay has been developed for apolipoprotein A1 (Apo-A1) utilizing a fluoroscein labeled affinity purified antibody and a capture antibody immobilized on an agarose gel, solid-phase support having a diameter size average of 200 µm The solid support and labeled antibody were lyophilized at -45^{o}C and tableted with an effervescing agent for ease of handling. The assay was performed by adding a 0.5 ml aliquot of a prediluted sample or calibrator in a buffer diluent of 0.15M Tris, 0.1M NaCl, 0.1% BSA and 0.1% Na azide to a test tube, addition of the tablet, and incubation for 10 minutes at 37^{o}. After incubation is complete, the same diluent buffer is added to remove any unbound labeled antibody from the solid-phase and the tube allowed to settle for 4 minutes before counting the absorbance in the supernatant for 1-2 seconds in a tube photometer. The absorbance in the supernatant which is unbound antibody is inversely proportional to the concentration of Apo-A1 in the patient's sample.

The dynamic range of the assay is 50 to 180 mg/dl and a 5 to 9% within assay variance has been shown over this range. The normal range for 64 males is 122 mg/dl ± 21 and for 62 females is 130 mg/dl ± 21. The assay has shown good correlation with turbidimetric reference procedure in normals and coronary risk patients. For 36 coronary risk patients a correlation coefficient (R) of 0.94 (Y=0.877+19.99) was obtained and for 88 normals an R of 0.81 (Y=0.973+10.71).

### Example 2

An immunoassay procedure for theophylline.

A dye-labeled monoclonal antibody is conjugated with theophylline and immobilized in a solid-phase tablet prepared as described in Example 1.

Serum or plasma sample is added to a reaction vial, followed by addition of the theophylline tablet, and a 10 minute incubation at 37^{o}. A counting tube containing the diluent buffer of Example 1 is inserted into the reaction vial, the tube assembly inverted three times, assembly allowed to settle for 4 minutes, and counted for 1-2 seconds in a tube photometer. Theophylline in the sample displaces the conjugate on the solid support such that absorbance thereof in the supernatant is a measure of the amount of theophylline in the sample. The assay is rapid, precise, accurate, and suited for on-site testing environments. Over the dynamic range of 2.5-30 µg/ml within-day precision studies showed CV's of 6-9% and between-day CV's of 5-6.1%.

Good correlation of serum or plasma was shown with an EMIT™ reference assay (Y=0.985x0.05,R=0.97) and TDX™ assay (Y=1.04x0.95,R=0.99).

## Claims

1. A heterogeneous immunoassay utilizing a solid-phase carrying at least one antigen or antibody and further utilizing a labeled antigen or antibody as appropriate, wherein a sample containing an analyte is contacted with said labeled antigen or antibody in the presence of said solid phase to produce an immunological complex on said solid-phase and an unbound labeled antigen or antibody, characterized by providing said solid-phase in the form of a tablet and mixing the thus contacted solid-phase in a diluent in which the unreacted labeled antigen or antibody is soluble.

2. The immunoassay according to claim 1 wherein the solid-phase is agarose gel.

3. The immunoassay according to claim 2 wherein the agarose gel has a particle size diameter in the range of 150 to 300 µm.

4. The immunoassay claim 3 wherein the diluent is a Tris buffer.

5. A kit for carrying out the immunoassay according to any of claims 1-4, comprising at least a reaction tube containing the diluent; the tablet containing the solid phase and the antigen or antibody; and a reading tube containing the diluent.

## Patentansprüche

1. Heterogener Immunoassay, bei dem eine Festphase verwendet wird, die mindestens ein Antigen oder einen Antikörper trägt, und bei dem darüber hinaus dem ensprechend ein markiertes Antigen oder Antikörper verwendet wird, wobei eine Probe, die einen Analyten enthält, mit dem markierten Antigen oder Antikörper in Gegenwart der festen Phase in Kontakt gebracht wird, um einen immunologischen Komplex auf der Festphase und ein ungebundenes markiertes Antigen oder Antikörper zu bilden, dadurch gekennzeichnet, daß die Festphase in Form einer Tablette bereitgestellt wird und die so kontaktierte Festphase in einem Verdünnungsmittel gemischt wird, in dem das nicht umgesetzte markierte Antigen oder Antikörper löslich ist.

2. Immunoassay nach Anspruch 1, bei dem die Festphase Agarosegel ist.

3. Immunoassay nach Anspruch 2, bei dem das Agarosegel einen Teilchengrößendurchmesser im Bereich von 150 bis 300 µm aufweist.

4. Immunoassay nach Anspruch 3, bei dem das Verdünnungsmittel ein Trispuffer ist.

5. Kit zum Durchführen des Immunoassays nach einem der Ansprüche 1 - 4, umfassend mindestens ein Reaktionsröhrchen, das das Verdünnungsmittel enthält; die Tablette, die die feste Phase und das Antigen oder den Antikörper enthält; und ein Ableseröhrchen, das das Verdünnungsmittel enthält.

## Revendications

1. Dosage immunologique hétérogène utilisant une phase solide portant au moins un antigène ou un anticorps et en outre utilisant un antigène ou un anticorps marqué selon la nécessité, dans lequel un échantillon contenant l'élément à analyser est mis en contact avec ledit antigène ou ledit anticorps marqué, en présence de ladite phase solide pour produire un complexe immunologique sur ladite phase solide et un antigène ou un anticorps marqué non lié, caractérisé en ce que ladite phase solide est fournie sous forme d'une tablette et en ce que la phase solide ainsi mise en contact est mélangée à un diluant dans lequel l'antigène ou l'anticorps marqué qui n'a pas réagi est soluble.

2. Dosage immunologique conforme à la revendication 1, dans lequel la phase solide est un gel d'agarose.

3. Dosage immunologique conforme à la revendication 2, dans lequel le gel d'agarose possède un diamètre de particules compris entre 150 et 300 µm.

4. Dosage immunologique conforme à la revendication 3, dans lequel le diluant est un tampon Tris.

5. Nécessaire pour effectuer le dosage immunologique conforme à une quelconque des revendications 1 à 4, comprenant au moins un tube de réaction contenant le diluant; la tablette contenant la phase solide et l'antigène ou l'anticorps; et un tube de lecture contenant le diluant.
